Europäisches Patentamt

European Patent Office

Office européen des brevets

(11) Publication number: **0 318 302**
**A1**

(12) **EUROPEAN PATENT APPLICATION**

(21) Application number: **88311158.5**

(22) Date of filing: **25.11.88**

(51) Int. Cl.⁴: **C 12 P 21/00**
C 12 R 1/19, A 61 K 37/02,
A 61 K 35/74, G 01 N 33/569,
G 01 N 33/68, G 01 N 33/574

(30) Priority: **27.11.87 US 127032**

(43) Date of publication of application:
**31.05.89 Bulletin 89/22**

(84) Designated Contracting States:
**AT BE CH DE ES FR GB GR IT LI LU NL SE**

(71) Applicant: **The University of Toronto Innovations Foundation**
**203 College Street, Suite 205**
**Toronto Ontario M5T 1P9 (CA)**

(72) Inventor: **Farkas-Himsley, Hannah**
**21 Edgar Avenue**
**Toronto Ontario, M4W 2B1 (CA)**

**Henson, Geoffrey Wyatt**
**81 Devon Drive**
**Exton, PA. 19341 (US)**

**Brooks, Robert Charles**
**792 Manatawna Avenue**
**Philadelphia, PA 19128 (US)**

(74) Representative: **Arthur, Bryan Edward et al**
**Withers & Rogers 4 Dyer's Buildings Holborn**
**London EC1N 2JT (GB)**

(54) **Proteinaceous compositions.**

(57) A protein or proteinaceous composition obtainable by cultivating bacteria cells in the presence of an inducer, such as an inducer of bacteriocin synthesis, and extracting and purifying the composition, is useful in detecting or killing the growth of virally infected or tumorigenic mammalian cells. The protein or proteinaceous composition may contain bacteriocin, or may be further purified to remove substantially all of the bacteriocin component. The proteinaceous composition substantially free of bacteriocin is able to kill tumorigenic cells.

EP 0 318 302 A1

## Description

This invention relates to protein and proteinaceous compositions exhibiting anti-tumor and/or anti-viral effects.

White blood cells comprise, among other components, T and B cells (lymphocytes). Of the viruses which are known to infect T-cells, the virus of extreme medical importance among these is HIV-I, a retrovirus, which has recently been identified as the virus responsible for Acquired Immune Deficiency Syndrome or AIDS. It has been shown that the HIV-I, involved in AIDS, can infect the $T_4$ cells in the blood and eventually destroy them. The $T_8$ suppressor cells are not subject to such infections.

Other medically important viral infections include infectious mononucleosis caused by the Epstein-Barr virus (EBV), and influenza caused by various influenza virus types.

The AIDS virus is of course a particularly acute medical problem currently, with no proven curative effective antidote yet being generally available. The AIDS virus may infect the T-cells of a patient's blood and lie dormant therein for extended periods of time. However, when some factor triggers their replication, they rapidly destroy the host T-cells, until they are incapable of performing their immune function against body invaders.

Researchers have previously identified and isolated bacterial-derived proteins now referred to as bacteriocins. These proteins are produced by a wide variety of bacteria, both Gram-positive and Gram-negative, and are named after the producer strain. Bacteriocins interact with specific bacterial strains normally closely related to the producer strain and prevent their multiplication by killing them. Bacteriocins are produced upon growth and subdivision of the bacterial cells in presence of an inducer which promotes the expression and/or secretion of a wide variety of proteins among which are the bacteriocin proteins.

It is an object of the present invention to provide a means for detecting virally infected mammalian cells.

It is a further object of this invention to provide a means for detecting malignant mammalian cells.

It is a further object of the present invention to provide substances and compositions which can be used in the therapy of various virally induced infections and diseases.

It is yet another object of this invention to provide substances and compositions which can be used in the therapy of malignant growths.

The present invention is based on the observation that a protein or proteinaceous compositions obtainable by cultivating bacterial cells in the presence of a bacteriocin-producing or stimulating inducer, and extracting and at least partially purifying the proteinanceous composition therefrom, exhibits antiviral and/or anti-tumor activity on mammalian cells. The protein or proteinaceous composition may contain bacteriocins. Whilst the composition first prepared in this manner contains bacteriocins and is active anti-virally and anti-tumorigenically, it can be further purified to remove substantially all of the bacteriocin component and the resultant protein or modified proteinaceous composition exhibits at least one such activity on mammalian cells in substantial amount.

As used herein, the term "malignant" means "having the property of locally invasive and destructive growth and metastasis", i.e. as defined in Stedman's Medical Dictionary, 24th Edition, William & Wilkins, Baltimore, London, 1982.

Accordingly, it should be understood that, as used herein, the term "virally infected mammalian cells" excludes virally-infected tumorigenic or malignant cells; namely the virally infected mammalian cells referred to herein have self-limiting growth which is, prima facie, uncharacteristic of malignant cells.

According to the present invention, therefore, one or more proteins and proteinaceous compositions are provided for use in treating virally infected mammalian cells and/or tumorigenic mammalian cells.

There is also provided, within the scope of this invention, a means for detecting the growth of virally infected non-malignant mammalian cells and malignant mammalian cells which comprises interacting these cells with the protein or proteinaceous composition of the present invention to hit selectively the virally infected or malignant cells.

The active ingredient of the proteinaceous compositions of the invention may be a single protein, or one or more proteins in an association. It has been found that the active ingredient(s) in these compositions selectively interact(s) with the virally infected cells or the malignant cells. Moreover, appropriate doses of protein or the proteinaceous compositions appear to kill selectively the virally infected cells or the malignant cells, as the case may be, whilst leaving the normal healthy cells substantially unaffected.

The proteinaceous compositions useful in the invention may contain a bacteriocin component. The compositions, according to one process of their preparation, are formed along with bacteriocins. Such bacteriocin preparations are, in many instances, the same as those which discriminately kill or inhibit the growth of various cancer cells such as fibrosarcoma, acute lymphoblastic leukemia (ALL), cervical carcinoma, adenocarcinoma, squamous cell carcinoma, chronic myelogenous leukemia, prolymphocytic leukemia, mastocytoma and many others. Specific examples of bacteriocins, which may be produced along with the active ingredient or ingredients of the present invention include pyocins, vibriocins, mycobacteriocins and colicins. Their residual presence does not appear to be harmful. However, proteinaceous compositions substantially free of any bacteriocins exhibit anti-tumor and/or anti-viral activity.

Virally infected, non-malignant, mammalian cells may be detected within this invention by treatment of these cells with the protein or proteinaceous compositions as described herein. More specifically, cells infected by

2

viruses can be detected by cell death of such cells treated with the protein or proteinaceous compositions as described herein.

In addition, malignant mammalian cells may be detected according to this invention by treatment of the cells with the protein or proteinaceous compositions as described herein. More specifically, malignant cells can be detected by cell death of such cells treated with the protein or proteinaceous compositions as described herein.

There are a number of methods known to those skilled in the art, that can effectively be used for detecting virally infected, non-malignant cells and malignant cells treated with the compositions of the present invention. These include, but are not limited to, binding assays (enzyme-linked, fluorescent and radiobinding), and metabolic uptake assays including the uptake of tritiated thymidine and flow-cytometry.

The proteinaceous compositions can be prepared by known, standard techniques of bacterial cultivation using a strain of bacteria which has the potential for bacteriocin production. The bacteria are grown in a fermenter to the exponential phase. Induction follows, by the use of a suitable chemical or physical agent, which will cause most of the cells to synthesize many proteins which had been repressed prior to inducer treatment. These cells (90 - 99% of them), under suitable induction conditions will also synthesize bacteriocin. An example of a suitable inducer is mitomycin C, at a level of about 0.5 micrograms per millilitre. Other inducers are known and widely applied in the art and include the application of UV light and suitable redox conditions. The cells are further incubated. In the case of some cultures, the proteins are secreted into the cultivation growth medium. In other cases, the cultures will not release the proteins so that the cell wall needs to be broken up. This may be done under pressure (20,000 psi, for example) using a French pressure cell. The impure proteinaceous composition may be separated from cell debris by centrifugation and is then contained in the supernatant liquid.

Having recovered the supernatant, standard biochemical separation processes are used to purify the proteinaceous composition. For example, a salt such as ammonium sulphate is preferably used at various concentrations, initially. Residual ammonium sulphate is then removed by dialysis against water. When the desired proteinaceous composition is to contain bacteriocin, the suspended precipitate containing the proteinaceous composition may be chromatographed on a column such as Sephadex DEAE-A50, a weak cation exchanger, and its various proteins separated by monitoring absorbance at 280nm. Active fractions can be determined from among the proteins thus separated, and selected on the basis of the efficacy with which aliquots thereof kill bacterial cells, i.e. the indicator strain, known to be sensitive to the bacteriocin component of the proteinaceous composition. Active protein fractions are then pooled. Further purification may be carried out by high performance liquid chromatography (HPLC) based on the charge of the proteins. The various peaks obtained by monitoring absorbance at 280 nm may be separated and again tested for activity against the indicator strain. Final purity of the proteinaceous composition may be determined by sodium dodecyl sulphate polyacrylamide gel electophoresis (SDS-PAGE).

When the desired final protein or proteinaceous composition is to be substantially free from bacteriocin, then a size exclusion column such as AcA54 may be used in place of the Sephadex DEAE-A50, followed by subjection of the active fraction, as meausred by bacteriocin activity, to ion exchange chromatography on, for example, a DEAE-TRISACRYL-M ion exchange column. This will separate the active protein or proteinaceous material from the bacteriocin. Now the active fraction is determined by activity against mammalian malignant cells, e.g. by tritiated thymidine uptake measurements. Then further purification of the active protein or proteinaceous material can be achieved by HPLC-SEC (size exclusion chromatography). The active fraction is again determined by activity against mammalian malignant cells.

Thus, the selection, isolation and purification of the proteinaceous composition for use in the present invention is within the skill of the art. Routine protein production and screening procedures as described above will yield to the skilled worker a protein or proteinaceous compositions which are active against virally infected mammalian cells and/or against tumorigenic mammalian cells.

The resultant proteinaceous composition has been demonstrated to be active against tumorigenic mammalian cells. It contains only trace residual amounts of bacteriocin, and shows minimal antibacterial activity. The separated bacteriocin-containing fraction, on the contrary shows strong antibacterial activity, as would be expected, but no significant anti-tumorigenic activity.

The proteinaceous composition useful in the present invention, containing unseparated bacteriocin, should be used in relatively small amounts, to safeguard against toxicity problems. In toxicity studies conducted on mice, the effective repeated dose of micrograms per animal was 100% effective without any toxic effects on the mice. However, with increase in concentrations of the composition, toxicity was apparent. With mice of strain C3H/J, 80 to 100 micrograms per mouse was fatal. With other species of mice, such as hybrid C3DX2F1/J, a 10-fold greater resistance than for C3H/J mice was noted, and toxicity was reached at 1,000 micrograms per mouse.

Suitable dosages of protein or proteinaceous composition for administration to mammals are in the range of from about 0.1 micrograms to 500 micrograms per kilogram body weight, preferably from about 5 to about 100 micrograms per kilogram body weight, of mammalian patient. Repeated doses of the size may be administered. It is preferred that the composition be added to a given lymphocyte population to arrive at a ratio of $10^{-5}$ ng to 10 ng proteinaceous composition per target cell, most preferably, $10^{-4}$ ng to 1 ng per target cell. The dosage size to kill virally-infected cells is significantly smaller than the dose required to treat cancerous cells.

The proteinaceous composition according to the invention which did not contain significant residual bacteriocin did not exhibit toxicity under therapeutic dosage, i.e. 10 micrograms per mouse.

Dosages and concentration for killing malignant cells with substantially bacteriocin-free composition are essentially similar to the dosages and concentrations of the proteinaceous composition containing unseparated bacteriocin described above. both for in vivo and in vitro testing.

The method of administration of the the proteins and proteinaceous compositions of the invention will influence the dosage form of the composition. Because the compositions comprise proteins and may be digested by stomach enzymes, and also because, preferably, the compositions are used in attack on lymphocytes, the compositions are preferably prepared as solutions for administration by injection. Accordingly, preferred carriers are buffered in saline aqueous media. Such solutions are formulated according to standard practice in the art. Other methods of administration suitable to ensure that the active material reaches the target site in its active condition may also be adopted, and will be determinable within the skill of the art. Examples of such administration forms include, but are not limited to, controlled or sustained release compositions.

The invention is further illustrated in the following specific examples:

Example 1 - Preparation of Proteinaceous Composition

To two hundred litres of Brain Heart Infusion medium (BHI) (Difco Labs, Detroit, Mich.) was added on inoculum of the bacterial producer strain E. coli HSC-10 which had been grown overnight. The culture was grown at 37° until the bacterial growth reached the exponential phase. An inducer, namely Mitomycin C in the amount of 0.5 micrograms per ml was then added to the culture and growth was allowed to continue for a further 4 - 5 hours.

The resulting broth was spun on a Sharples centrifuge, for ninety minutes, to form a supernatant broth which was discarded, and a pellet of solid and semi-solid material. The pellet had a wet mass of 505g. The cellular material within the pellet was then disrupted using a French pressure cell and the resultant material was resuspended in 1% of its original volume of Tris (0.05M, ph 7.8). The resulting suspension was spun, and the pellet of debris discarded. The supernatant material so obtained, containing the lysate which contained the crude proteinaceous composition, was worked up and purified as described below in Example 2.

Example 2 - Purification of Proteinaceous Composition

The supernatant containing the crude proteinaceous composition obtained in Example 1 (i.e. the crude lysate, composition 1) was freed from proteins other than the bacteriocidally active components by ammonium sulphate precipitation at various concentrations. Bacteriocin was present in the proteinaceous compositions obtained, as evidenced by bacteriocidal activity thereof.

The protein which salted out at a concentration of 35-50% of ammonium sulphate was retained, and the supernatant discarded. The precipitate retained material was resuspended, dialysed against water and spun at 46,000xg, to yield proteinaceous composition 2. This resultant supernatant was stored at -20°C prior to subsequent purification.

After thawing, the frozen supernatant was subjected to chromatography using Sephadex - DEAE A-50 and collected in separate fractions using a gradient of 0.0 - 1.0M NaCl in 0.01M Tris, pH 8.2 as eluant and monitoring absorbance at 280nm.

Fractions of interest were then pooled to yield the proteinaceous composition 3, hereinafter HSC-10, and tested for bacteriocidal activity against the indicator strain E. Coli Y10 using the turbidity test. Specifically, the indicator strain grown overnight in brain heart infusion medium at 37°C was used as inoculum to obtain exponential growth to a specific cell number. These bacteria were mixed with two-fold dilutions of the partially purified proteinaceous composition as described above and growth inhibition of the indicator strain was recorded spectrophotometrically. Growth response curves were generated by these turbidity measurements from which the lethal units/ml of the various compositions were calculated in view of the known concentration of proteinaceous composition added to the known number of cells of the indicator strain in the medium. The LU50, lethal units per ml of the various compositions required to kill 50% of the indicator strain population, is shown in and discussed with reference to Table 1 hereinafter,

Further purification of the pooled fractions was carried out using high performance liquid chromatography (HPLC) and those fractions collected which exhibited an absorbance peak of 280 nm, indicating proteinaceous nature were pooled to yield proteinaceous composition 4, and aliquots thereof subjected to the same turbidity test as described above.

The amounts of bacteriocidally active proteinaceous material recovered at each step in the method described above and the bacteriocidal activity of those amounts is recorded in Table 1 appearing below:

TABLE I

Purification Data for Colicin $HSC_{10}$

| Test Material | PROTEINACEOUS CONTENT | | BACTERIOCIDAL POTENCY(a) | |
|---|---|---|---|---|
| | Total (mg) | % of original | $LU_{50}$ (ug/ml) | Specific activity |
| 1. Crude Lysate | 15,674 | 100 | 0.67 | $4.3 \times 10^{-5}$ |
| 2. PPT. 35-50 (percent cut) | 1,298 | 8.28 | 0.56 | $4.3 \times 10^{-4}$ |
| 3. PPT. 35-50% after Sephadex DEAE - A50 ("HSC 10") | 139.8 | .89 | 0.16 | $1.1 \times 10^{-3}$ |
| 4. Active Material from Sephadex after HPLC - DEAE | 15.9 | .10 | 0.63 | $4.0 \times 10^{-2}$ |

a) Tested against bacteria by turbidity test.

It will be noted that the amount of active protein derived from the culture lysate is only about 0.10% of the original weight of the lysate, suggesting that, preferably, large volumes of bacteria are cultured in order to generate necessary amounts of proteinaceous composition. The method of purification preferred herein was sufficient to establish a purification factor, based on the bacteriocidal activity of a protein which is a component of the proteinaceous composition, of 930 at which only 0.63 ug of the proteinaceous composition is required to inhibit growth of 50% of the indicator strain population. The fraction that is isolated for further study in Examples 4, 5 and 6, and referred to hereinbelow as HSC-10 is test material 3 from Table I.

Example 3 - Preparation of Proteinaceous Material Substantially Free from Bacteriocin
Protein concentrations were determined by the Coomassie Brilliant Blue G250 dye binding assay of Bradford (Bio-Rad). Water used in all solutions was distilled and passed through a Nanopure system (Millipore) and had a conductance value of 18.3 ohms/cm.

A portion of the 35-50% ammonium sulfate fraction of E. coli HSC10 crude cellular lysate (fraction 2 of Table 1, Example 2) in 20 mM TRIS-HCL pH7.4 was thawed and treated immediately with 100 mM phenylmethane sulfonyl fluoride [PMSF] (Sigma) in 95% ethanol (Pharmaco), to inhibit protease activity, such that the final concentration of PMSF in solution was 1 mM.

The PMSF treated proteins (1 to 1.5 g/30 to 35 mL) were loaded onto a 95 x 2.6 cm low pressure chromatography column (LKB-Pharmacia) packed with 450 mL of AcA54 (IBF Products) and equilibrated with 50 mM TRIS-HCl pH7.4 (LKB Ultrograde). The proteins were eluted isocratically with the equilibration buffer at a flow rate of 40 mL/hr (ISCO Tris Pump). The eluent was monitored at 280 nm (0 to 2 AUFS) (ISCO UA-5 Absorbance/Fluorescence Detector) and 6 mL fractions were collected (ISCO Retriever II Fraction Collector).

The active fractions were identified by assaying for the antibacterial action of Bacteriocin HSC10 which coelutes from the AcA54 column with the cytotoxic protein of interest. These fractions showing inhibition of E. colii Y10 growth at 1/1000 or greater dilutions were pooled and dialyzed in Spectra/Por3 tubing (Spectrum Medical Industries) for 24 hrs against 4 L of water. The dialyzed material was lyophilized and the pellet resuspended in water.

The resuspended protein was loaded onto a 25 x 2.5 cm low pressure column (Altex) packed with 100 mL of DEAE-TRISACRYL-M (IBF Products) equilibrated with 20 mM TRIS-HCl pH8.0 (LKB Ultrograde). Immediately after loading the flow was stopped for 15 minutes to allow for protein binding to the packing material. The column was then developed for 1 hour isocratically with the equilibration buffer followed by a linear gradient of 0 to 0.35 M sodium chloride (Aldrich 99 + %) in the equilibration buffer at a flow rate of 40 mL/hr (ISCO Tris Pump). The elution was monitored at 280 nm (0 to 0.5 AUFS) (ISCO UA-5 Absorbance/Fluorescence Detector) and 6 mL fraction were collected (ISCO Retriever II Fraction Collector). The sodium chloride concentration of the gradient was monitored with a YSI Model 32 Conductance Meter and a Beckman Industrial Flow cell calibrated with a series of 0, 0.1, 0.2, 0.3, 0.4, 0.6, and 0.8M sodium chloride/20 mM TRIS-HCl pH = 8.0 standards (LKB-Ultrograde).

Bacteriocin HSC10 containing fractions were identified by bacterial spot testing. Those fractions showing E. coli Y10 growth inhibition at 1/1000 or greater dilutions were pooled, dialyzed against 2 L of 50 mM ammonium bicarbonate (Fisher Cert.) and stored, lyophilized at -20°C.

Active fractions at 1 ng protein per cell were assayed for anti-tumorigenic activity by interaction with HL-60 human myelogenic cells (ATCC) for 24 hour incubation at 37°C, 5% $Co_2$ (Matheson) in RPMI 1640 (GIBCO) with 10% FCS (GIBCO) at 1 ng/cell. $^3$H-thymidine was then added for 4 hours while continuing incubation as above. Fractions inhibiting over 75% were pooled and dialyzed against 4 L of 50 mM ammonium bicarbonate for 24 hours (Fisher Cert.). The samples were lyophilized and taken up in phosphate buffered saline (PBS) at pH 6.9, 0.2 g potassium chloride (Aldrich 99 + %), 0.2 g monobasic potassium phosphate (Aldrich 99 + %), 2.16 g dibasic sodium phosphate (Aldrich 99 + %), 8 g sodium chloride (Aldrich 99 + %)/Litre Nanopure water pH with concentrated HCl (Fisher ACS reagent).

The same pooled active fractions were also subjected to antibacterial activity tests, to quantify the residual bacteriocin therein. It was found that a concentration of 21.4 micrograms per ml of this fraction was necessary to inhibit 50% bacterial growth, as compared with a concentration of 0.12 micrograms per ml of the material prior to DEAE-TRISACRYL-M column treatment.

The resuspended proteins (20 to 40 mg) were injected onto a 60 x 2.15 cm TSK G3000SW HPLC size exclusion column (Phenomenex equilibrated wtih PBS, pH 6.9. (HPLC system refrigerated Water Wisp Auto-injector, LKB 2152 HPLC controller, 2 LKB 2150 HPLC titanium pumps LKB 2210 2-channel recorder, LKB 2151 variable wavelength detector and LKB 2211 suprac fraction collector. This system separates proteins by molecular weight. Proteins of molecular weights in the approximate range 3,000-150,000 were eluted isocratically with the equilibration buffer at a flow rate of 3.5 mL/min (25 bar pressure) and were monitored at 280 nm (0-0.16 AUFS) while collecting 7 mL fractions.

Active fractions were identified by $^3$H-thymidine assay, pooled, dialyzed against 2 L of 50 mM ammonium bicarbonate (Fisher Cert) for 24 hours and lyophilized.

Prior to use, the column was calibrated, using standard proteins of known molecular weight (Aldridge). The proteinaceous composition which eluted off the column had a molecular weight approximately 70,000 according to the standard calibration experiments. This composition was found to have high anti-tumorigenic activity. More specifically, this composition exhibited an $IC_{50}$ (concentration which inhibits 50% of tumorigenic cell growth) of 0.3 nanograms per cell. This activity test was conducted as previously described, by determining the uptake of tritiated thymidine by tumorigenic human lymphocyte cells.

Example 4 - Selectivity of Colicin HSC-10 in Identifying Virally Infected Leukocytes in Vitro

Lymphocytes obtained from 14 different AIDS patients were enriched on Ficoll-Paque® (Pharmacia Fine Chemical, Dorval, Quebec), counted and divided into experimental and control groups. Colicin HSC10, extracted and purified as described above, was diluted in tris buffer at pH 7.4 and added to the experimental lymphocyte population to arrive at a ratio of $10^{-5}$ to $10^{-2}$ ng HSC-10 per target cell. The control population received only the tris-buffer diluent.

The two cell populations were distributed into microwells and grown overnight in the presence of $^3$H-thymidine at 37° C in the presence of 5% $CO_2$ humidified air. Thereafter, the cells were harvested, washed free of excess $^3$H-thymidine and passed through a scintillation counter to evaluate uptake of radioactivity which is a reflection of cell metabolism and growth. The effect of HSC-10 was evaluated by % reduction of disintegrations per minute (dpm) in the HSC-10 treated group as compared to the control groups.

In a parallel experiment, HSC-10 was added to lymphocytes obtained from 9 healthy individuals and compared, via the $^3$H-thymidine uptake test, with control healthy lymphocytes subjected only to the tris-buffer which was used as a diluent for the HSC-10.

A third group comprising three patients in high-risk AIDS group (e.g. haemophiliacs) were tested, although the presence of AIDS in these patients was not confirmed. These patients were confirmed to be healthy by testing one and one half years later.

$^3$H-thymidine uptake in each of the three groups was measured as a percent of the control group, which was, in each case, patients or normals in the respective groups without HSC-10 treatment.

In the concentrations applied, the HSC-10 exhibited effects on the number of HIV-I infected (AIDS) lymphocytes from the AIDS patients or normals, and on the lymphocytes from the AIDS suspects and the healthy, normal individuals as shown by Table II below:

## TABLE II

| | $^3$H-thymidine uptake as a % of control | | Number of test results in each category |
|---|---|---|---|
| | range | average | |
| **AIDS Patients** | | | |
| 15 tests (on 14 different patients) | | | |
| Inhibition | 20-86 | 53 | 8 out of 15 (53%) (7 out of 14 patients) |
| Borderline Inhibition | 99-105 | ·102 | 2 out of 15 (13%) (2 out of 14 patients) |
| Stimulation | >110 | 270 | 5 out of 15 (33%) (5 out of 14 patients) |
| **AIDS Suspected** | | | |
| 3 tests (on 3 different suspected AIDS patients) | | | |
| Inhibition | - | | 0 out of 3 |
| Borderline Inhibition (90-110) | 100-111 | 105 | 2 out of 3 |
| Stimulation | >110 | 145 | 1 out of 3 |
| **Normals** | | | |
| 14 tests (on 9 different individuals) | | | |
| Inhibition | 43-67 | 51 | *4 out of 14 (29%) (4 out of 9 individuals) |
| Borderline Inhibition (90-110) | 90-95 | 93 | 2 out of 14 (14%) (2 out of 9 individuals) |
| Stimulation | >110 | 166 | 8 out of 14 (57%) (7 out of 9 individuals) |

*As described hereinabove, four tests from samples from individuals in the "Normal" group, known not to have the AIDS virus, were sensitive to HSC-10 in the $^3$H thymidine inhibition test, which is indicative of a viral infection. All of these "false-positive" individuals were later confirmed as having other viral infections by $^3$H-thymidine uptake and two individuals by the 2-5 A synthetase test. This latter test, described in Journal of Infectious Diseases, Read, S.E., et al Sept./85, is widely known and accepted in the art for the early diagnosis of viral infections. In a later study, in accordance with a transient viral infection, one of the "false-positive" patients exhibited stimulation, as opposed to inhibition of $^3$H-thymidine.

Of the fifteen tests performed on the AIDS patients, 53% showed an inhibition of thymidine uptake, with 13% showing borderline inhibition. Conversely, of the fourteen tests performed on the normal individuals, 57% showed an effective stimulation of thymidine uptake, with 14% showing borderline inhibition, and only 29% (4 out of 14) showing inhibition.

None of the three suspected AIDS patients showed sensitivity to HSC-10. At the time of this testing, tests and observations confirmed that none of these patients carried the AIDS virus.

In summary, a remarkable effect can be observed from the results presented in Table II. Firstly, the number of AIDS lymphocytes exhibiting uptake of the radioactivity is significantly reduced when HSC-10 is introduced. Secondly, and importantly, growth of normal lymphocytes subjected to the same concentrations of HSC-10 appears not to be impaired (71%). Still more interestingly, 57% of those normal lymphocytes which were subjected to HSC-10 exhibited an increase in radioactivity count by comparison with those untreated. This signifies stimulation of growth of normal lymphocytes, a response which will presumably allow a substantial number of patients treated with HSC-10 greater immunity against other infections which attend AIDS patients.

To identify the target of HSC-10 attack on AIDS infected lymphocytes more specifically, monoclonal antibodies were employed. It is known that the ratio of sub-set helper-inducer ($T_4$ cells to sub-set suppressor ($T_8$) cells which interfere with the immune response of the AIDS patient, i.e. the $T_4/T_8$ ratio, is reduced in AIDS patients - see Evatt et al., New England Journal of Medicine, 1985 Vol. 313, page 483. The subset helper-inducer cells ($T_4$) which activate the immune responses and are therefore vital components of the body defenses, are infected with HIV-I viruses and their function is impaired or non-existent. Experiments were therefore carried out in which specific monoclonal antibodies to $T_4$ cells (New England Nuclear, Catalogue no. NEN-038) and to $T_8$ cells (New England Nuclear, Catalogue no. NEI-039) were used to evaluate the presence and numbers of $T_4$ and $T_8$ lymphocytes after HSC-10 treatment of lymphocytes derived from an AIDS patient.

It was found that the numbers of subset $T_8$ suppressor lymphocytes remained unchanged after HSC-10 treatment whereas the number of the infected $T_4$ cells were significantly reduced.

Specifically, the test was performed using specific monoclonal antibodies to $T_4$ and $T_8$ cells, described above, prepared by a process entitled the Hybridoma Technique. This technique is widely applied by those skilled in the art.

The monoclonal antibodies to $T_4$ or $T_8$, thus prepared, are labelled with a fluorescent compound (e.g. fluorescein isothiocyanate) which may be detected due to the fluorescence of the dye under ultraviolet light.

The $T_4$ and $T_8$ cells are then associated with their respective fluorescent antibodies and counted under ultraviolet light.

Each of the AIDS patients tested showed in the presence of HSC-10 a decrease of between 14-21% in their $T_4$ lymphocytes. Their $T_8$ lymphocytes were either not affected at all or nominally stimulated or inhibited.

Thus according to the specific embodiment described herein, it has been shown that the proteinaceous composition, HSC-10, is effective in reducing the number of virally infected T-lymphoctyes. More specifically, the utility of this composition is established in reducing the number of $T_4$ cells without corresponding harm but rather stimulation to normal T cells.

Example 5

The effect of HSC-10 on AIDS patients lymphocytes as well as lymphocytes infected with other viruses such as sore throat influenza and infectious mononucleosis can be determined by flow-cytometry using techniques similar to those disclosed in IRCS Medical Science, 11, 236-237 (1983).

In general, the method involves analysis of the DNA content of each cell in a given sample in order to assess the percentage of cells existing, at an instant, in a specific phase of the cell cycle. Since HSC-10 can affect sensitive cells by degradation of DNA resulting in nucleotide leakage, an increasing number of treated cells with little DNA can be detected by flow-cytometry. Cells sensitive to HSC-10 and treated therewith will commonly be seen to accumulate within the "pre-$G_1$" channels when the results are plotted on a histogram which signifies decreasing DNA content, since the DNA of these cells is degraded.

More specifically, lymphocytes obtained from patients previously diagnosed as having infectious mononucleosis (by infection with the Epstein-Barr virus) were isolated by Ficoll-Paque (Pharmacia Fine Chemicals, Montreal) and stained with propidium iodide. HSC-10, extracted and purified as in Example 1, was added to a sample of the isolated lymphocytes. Cell fluorescence was then measured during the growth cycle in a flow cytometer (FCM). Lymphocytes to be tested, to which no HSC-10 had been added, were used to calibrate the $G_0/G_1$ phase cells to that of the standard. The total cell number tested was obtained from numerical data print out. The percent of the total was calculated for the cells present in the "pre-$G_1$" and $G_0/G_1$ phases by integration of the cells under the peak. The final results were expressed following subtraction or addition of the percent change which occurred in the cell numbers of the controls i.e. the virally infected cells not exposed to HSC-10.

The results are presented in Table III.

TABLE III

| Patient # | Test # | "Pre-$G_1$" | cells in Peak | $G_0/G_1$ | $SG_2M$ | Sensitive |
|---|---|---|---|---|---|---|
| 1 | 1 | +33 | -14 | -31 | -2.0 | + |
| | 2 | +24 | +17 | -24 | +0.2 | + |
| 2 | 3 | +17 | +11 | -18 | +1.0 | + |
| | 4 | +21 | +30 | -22 | +1.3 | + |
| 3 | 5 | +15 | +14 | -13 | -2.6 | + |
| 4 | 6 | -2.4 | +13 | -5.1 | +7.6 | + |
| 5 | 7 | +0.5 | +4.6 | -0.8 | +0.3 | - |
| 6 | 8 | -4.3 | +7.5 | +2.0 | +2.2 | ± |
| 7 | 9 | +1.3 | -1.1 | -0.3 | -1.2 | - |

There were nine tests performed on seven individuals. Five out of seven patients exhibited an accumulation of cells in the "pre-$G_1$" phase, thus showing sensitivity of the cells to HSC-10 (refer to column 3). Four of the seven patients tested showed on their histograms a decrease in cells from the $G_0/G_1$ phase (refer to column 5). In summary, in 78% of the tests, patient-samples exhibited sensitivity to HSC-10.

Example 6

Four patients categorized as Normal in the first section of Example 4 and who appeared to have the flu by clinical symptoms, were found to have a transiently positive reaction with HSC-10. These patients were subsequently tested positive for the presence of 2-5A synthetase, the presence of which is indicative of a viral infection (J. Infectious Diseases, Read, S.E., et al, Sept./85). After a period of time sufficient for recovery from the flu symptoms, these patients were retested for HSC-10 sensitivity and found to be negative. A tentative conclusion that can be drawn from these results is that at least some viral infections are affected by HSC-10.

Example 7 - In Vivo Testing of the Substantially Bacteriocin Free Proteinaceous Composition

The effect of the substantially bacteriocin free proteinaceous composition, as prepared in example 3, on tumor cells in mice was investigated. Fifteen 20-25 gram mice (C3H/J) were each injected intravenously with $10^4$ metasticizing cells. An experimental group of 5 mice were then injected intraperitoneally with 10 ug of the proteinaceous composition substantially free of bacteriocin of the present invention. A second experimental group of 5 mice were injected with 10 ug of HSC-10. The control group of 5 mice received only the buffer. The cells were allowed to grow for 3 weeks at which time the mice were killed. The lungs from all 15 mice were removed and the foci were counted. Each focus represents the growth of one cancerous cell. The percent difference in the number of foci between the control group and the treatment group is the percent inhibition of the cancer cells by HSC-10 or by proteinaceous composition substantially free of bacteriocin. There was a greater than 90% reduction of the foci in both experimental groups when compared to control.

Example 8 - In Vitro Testing of the Substantially Bacteriocin Free Proteinaceous Composition

Cancer cells from the myelogenic cell line HL-60 were grown on RPMI-1640 (Difco) medium and supplemented with 10% fetal calf serum (Difco) and 5% $CO_2$ at 37°C. The cells were then washed and suspended in fresh medium, counted and divided into experimental and control groups. Each group or population comprised approximately $4 \times 10^5$ cells. The proteinaceous composition, prepared as described in Example 3 above, was diluted in tris-buffer at pH 7.4 and added to the experimental cell population to arrive at a ratio of 2, 1.5, 0.5 and 0.25 ng protein per cell. The control population received only the tris-buffer diluent. The two cell populations were distributed into microwells at $8 \times 10^4$ cells and grown overnight in the presence of $^3$-H-thymidine at 37°C in the presence of 5% $CO_2$ humidified air. Thereafter, the cells were harvested, washed free of excess $^3$H-thymidine and counted in a scintillation counter to evaluate uptake of radioactivity which is a reflection of cell metabolism and growth. The effect of the modified proteinaceous composition was evaluated by a % reduction of disintegrations per minute (DPM) in the treated groups as opposed to the control groups.

The % difference between the DPMs of the controls and the DPMs of the experimental population is an indication of the effectiveness of the modified proteinaceous composition at killing malignant cells. `

It was found that less than 0.3 ng protein per cell of the modified proteinaceous composition substantially free of bacteriocin was sufficient to kill 50% of the cell population.

The results reported and presented herein indicate that the test compositions contain one or more proteins which are anti-tumorigenic and probably also anti-viral, and capable of combatting viruses such as HIV-I. The protein or proteins constituting the active ingredient or ingredients can be produced by inducing protein-expression and secretion by bacterial cells, under the influence of inducers commonly used to induce bacteriocin formation. The results above show that the active protein or proteins, both anti-viral and anti-tumorigenic, are present along with the bacteriocin protein when produced by such a bacterial cell cultivation methods, e.g. in compositions such as HSC-10. Anti-tumorigenic activity is demonstrated to be present in the proteinaceous composition from which the bacteriocins have been substantially removed.

Whilst the presently preferred process of preparing the anti-virally active or anti-tumorigenically active protein or proteins of the present invention is as described herein, namely by cultivation of appropriate bacterial cells under bacteriocin-inducing conditions, isolation of the proteinaceous compositions so produced, separation of bacteriocins therefrom, recovery of individual proteins from the resultant proteinaceous compositions, and testing by in vitro and/or in vivo methods to identify anti-tumorigenic and/or anti-viral proteins therefrom, it is anticipated that other processes of preparation may be developed. Once the routine purification to isolate individual proteins, and their routine testing for activity has been concluded, the proteins of interest can be synthesized by other known methods appropriate for their determined characteristics. Thus, from study of their amino acid sequences, routine stepwise synthesis of them by sequential coupling of amino acids, or short peptide chains of appropriate sequence, can be conducted, e.g. using the solid state Merrifield technique in which a solid activated gel is used as the support. Alternatively, from the knowledge of the amino acid sequence of the protein or proteins of interest, genetic engineering techniques can be devised and operated. Thus appropriate sequences of DNA can be inserted via a recombinant plasmid into an expression vehicle, e.g. any expression system compatible with the protein to the expressed, all according to known techniques within the skill of the art. The active protein or proteins so produced can be compared for identity with those produced by the methods described in detail and exemplified herein, to ensure that essentially the same materials have been produced.

Thus the protein or protein compositions of the invention, whilst obtainable along with bacteriocins by induced bacterial cell fermentation as described, are capable of production by other independent methods. They constitute distinct, novel compositions of matter, independent of their method of production.

**Claims**

1. A proteinaceous composition having at least one of anti-viral and anti-tumorigenic activity, said composition consisting essentially of at least one protein, and being obtainable by a process involving cultivating bacterial cells in the presence of an inducer, extracting the proteinaceous mixture from the cultivation medium, and recovering therefrom the resultant active proteinaceous composition so obtained.

2. The composition of claim 1, which is substantially free of bacteriocin.

3. The composition of claim 2 further characterized by being elutable from a size separation chromatography column at the position corresponding to that of elution of a protein of molecular weight approximately 70,00 daltons.

4. The composition of claim 2 which is obtainable by said process wherein the inducer is an inducer of bacteriocin synthesis.

5. The composition of claim 4 which is obtainable by said process in which the bacteriocin synthesized is selected from pyocins, vibriocins, mycobacteriocins and colicins.

6. The composition of claim 5 which is obtainable by said processes in which the inducer is mitomycin C.

7. The composition of claim 1 which is obtainable by said process wherein the bacterial cells are E. coli cells.

8. The composition of claim 1 including a bacteriocin, selected from pyocins, vibriocins, mycobacteriocins and colicins.

9. A method of inhibiting the growth of virally infected or malignant mammalian cells which comprises treating said cells with a growth inhibiting and/or cidal amount of the proteinaceous composition of claim 1.

10. A method of inhibiting the growth of malignant mammalian cells which comprises treating said cells with a growth inhibiting and/or cidal amount of the proteinaceous composition of claim 2.

11. The method according to claim 9 wherein said virally infected mammalian cells are white blood cells infected with a virus selected from HIV-I virus, the infectious mononucleosis virus and the influenza virus.

12. The method according to claim 11 wherein said virally infected mammalian cells are T-cells infected with the HIV-I virus.

13. The method according to claim 9 wherein the living mammal is administered a dose of the proteinaceous composition in the approximate range of 0.1 micrograms to 500 micrograms per kilogram of body weight.

14. The method according to claim 13 wherein the proteinaceous composition is administered in the approximate range of 5 micrograms to 100 micrograms per kilogram of body weight.

15. The method according to claim 10 wherein the living mammal is administered a dose of the proteinaceous composition in the approximate range of 0.1 micrograms to 500 micrograms per kilogram of body weight.

16. The method according to claim 15 wherein the proteinaceous composition is administered in the approximate range of 5 micrograms to 100 micrograms per kilogram of body weight.

17. The method according to claim 9 wherein the cells are present in a biological sample obtained from a mammalian body and are treated with an amount of the proteinaceous composition in the approximate range of $10^{-5}$ nanogram to 10 nanograms per cell.

18. The method according to claim 10 wherein the cells are present in a biological sample obtained from a mammalian body and are treated with an amount of the proteinaceous composition in the approximate range of $10^{-5}$ nanogram to 10 nanograms per cell.

19. A pharmaceutical composition useful in inhibiting the growth of and killing malignant mammalian cells and/or virally infected mammalian cells which comprises a proteinaceous composition as defined in claim 1 in admixture with a pharmaceutically acceptable diluent.

20. A pharmaceutical composition useful in inhibiting the growth of and killing malignant mammalian cells which comprises administering thereto an effective amount of the proteinaceous composition as defined in claim 2.

21. A method of treating a patient having a white blood cell viral infection selected from AIDS infections, infectious mononucleosis and influenza viral infection which comprises administering thereto an effective amount of the proteinaceous composition as defined in claim 1.

22. A method of detecting virally-infected non-malignant mammalian cells by the interaction of said cells with the proteinaceous composition of claim 1.

23. A method of detecting malignant mammalian cells by the interaction of said cells with the proteinaceous composition of claim 2.

European Patent
Office

**PARTIAL EUROPEAN SEARCH REPORT**
which under Rule 45 of the European Patent Convention
shall be considered, for the purposes of subsequent
proceedings, as the European search report

Application number

EP 88 31 1158

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 103, no. 15, October 14, 1985, page 33, abstract 115788a Columbus, Ohio, US H. FARKAS-HIMSLEY et al: "Purified colicin as cytotoxic agent of neoplasia: comparative study with crude colicin" & CYTOBIOS. 1985, 42(167-168), 193-207 <br><br> * Abstract * | 1,5-9, 17,19 | C 12 P 21/00 <br> C 12 R 1:19 <br> A 61 K 37/02 <br> A 61 K 35/74 <br> G 01 N 33/569 <br> G 01 N 33/68 <br> G 01 N 33/574 |
| X | CHEMICAL ABSTRACTS, vol. 106, no. 25, June 22, 1987, page 520, abstract 212336w Columbus, Ohio, US M. MITTLEMAN et al.: "Recognition of T-cell murine leukemia by bacteriocin (colicin); correlation with transplantation experiments" & Leuk. Res. 1987, 11(3), 215-22 <br><br> * Abstract * | 1,5-9, 17,19, 22 | **TECHNICAL FIELDS SEARCHED (Int Cl 4)** <br><br> C 12 P <br> A 61 K |

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely: 1-12,17-20,22,23

Claims searched incompletely:

Claims not searched: 13-16,21

Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 25-02-1989 | SKELLY |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1505.1 03 82

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 98, no. 25, June 20, 1983, page 289, abstract 212409p Columbus, Ohio, US E. MUSCLOW et al. "Bacteriocin and flow cytometry in laboratory diagnosis of leukemic peripheral blood lymphocytes and bone marrow cells" & EUR.J.CANCER CLIN. ONCOL. 1983, 19(2), 163-71 * Abstract * | 22 | |
| P,X | EP-A-0 247 873 (UNIVERSITY OF TORONTO) * Whole document * | 1,5-9, 11,12, 17,19, 22 | TECHNICAL FIELDS SEARCHED (Int Cl 4) |
| A | CHEMICAL ABSTRACTS, vol. 73, no. 10, September 7, 1970, page 101, abstract 53355h, Columbus, Ohio, US P.S. MORAHAN et al. "Age-related cellular resistance of the chicken embryo to viral infections. III. Escherichia coli resistance-inducing protein" & PROC. SOC. EXP. BIOL. MED. 1970, 134(1), 342-7 * Abstract * | 1 | |
| A | MICROBIOLOGICAL abstracts, section B, vol. 6, no. 6, March 1971 abstract B 4348 T. WATANABE et al.: "The antitumor action of bacterial extract. I. Suppression of mouse ascites tumors by the treatments with extracts of E. coli" & JAP. J. EXP. MED. vol. 39, 631-647 (1969) * Abstract * | 1 | |

./.

EPO Form 1505.3 06.78

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | BIOLOGICAL ABSTRACTS, vol. 66, November 15, 1978 · S. NAMIKI et al. : " Chemical and biological properties of the carcinostatic protein fraction of Escherichia coli" & GANN 69(2), 151-158, 1978 <br><br> * Abstract * | 1 | |
| A | PROC. SOC. EXPTL. BIOL. & MED. vol. 120, no. 3, 1965, pages 607-611 Y. CENTIFANTO: "A therapeutic antiviral from an extract of lambda infected E. coli (Phagicin)" | | TECHNICAL FIELDS SEARCHED (Int Cl 4) |
| A | FR-A-2 550 707 (LIPHA) | | |
| A | EP-A-0 213 811 (JOHNSON MATTHEY) | | |